# EUROPÄISCHE PATENTANMELDUNG

(11) **EP 3 922 985 A1**
(43) Veröffentlichungstag der Anmeldung: **15.12.2021**
(21) Anmeldenummer: 20179824.6
(22) Anmeldetag: 12.06.2020
(51) Int. Cl.: G01N 22/00, G01R 27/26, G01N 33/44

(54) **VERFAHREN UND VORRICHTUNG ZUR ÜBERWACHUNG DES REAKTIONSFORTSCHRITTS EINER POLYMERISATIONSREAKTION MITTELS MIKROWELLEN- ODER SUBMILLIMETERWELLENSTRAHLUNG**

(71) Anmelder: Covestro Deutschland AG, 51373 Leverkusen (DE)
(72) Erfinder: ZAREBA, Sebastian, 51107 Köln (DE); FRANKEN, Klaus, 51467 Bergisch Gladbach (DE); LYDING, Andreas, 47057 Duisburg (DE); WELSCH, Nicole, 51067 Köln (DE)
(74) Vertreter: Levpat

(57) **Zusammenfassung**

Die vorliegende Erfindung betrifft ein Verfahren zur Bestimmung des Reaktionsfortschritts einer Polymerisationsreaktion mittels Mikrowellen- oder Submillimeterstrahlung. Das erfindungsgemäß Verfahren umfasst folgende Verfahrensschritte:
- Bereitstellen einer Fläche (6) zur Aufnahme eines Reaktionsgemisches (2) für eine Polymerisationsreaktion,
- Bereitstellen einer Sendeeinheit (1) und einer Empfangseinheit (1) für Mikrowellen- oder Submillimeterwellenstrahlung, wobei die Fläche (6) bezogen auf den Strahlverlauf zwischen Sendeeinheit (1) und Empfangseinheit (1) angeordnet wird, wobei der Abstand zwischen Sendeeinheit (1) und Empfangseinheit (1) bestimmt wird und wobei die Sendeeinheit (1) und die Empfangseinheit (1) mit einer Auswerteeinheit verbunden sind,
- Aufbringen eines Reaktionsgemisches (2) auf die Fläche,
- wiederholtes Durchstrahlen des Reaktionsgemisches (2) mit FMCWmodulierter Mikrowellen- oder Submillimeterwellenstrahlung mittels der Sendeeinheit (1),
- Empfangen der Mikrowellen- oder Submillimeterwellenstrahlung nach Durchgang durch das Reaktionsgemisch (2) mittels der Empfangseinheit (1),
- Bestimmung der Laufzeit und/oder der Signalschwächung der Mikrowellen- oder Submillimeterwellenstrahlung von der Sendeeinheit (1) zur Empfangseinheit (1) in der Auswerteeinheit zur Überwachung des Reaktionsfortschrittes der Polymerisationsreaktion.

Ferner betrifft die Erfindung eine Vorrichtung zur Durchführung des Verfahrens

## Beschreibung

Die Erfindung betrifft ein Verfahren zur Überwachung des Reaktionsfortschritts einer Polymerisationsreaktion, insbesondere zur Herstellung von Polyurethanen, mittels Mikrowellen- oder Submillimeterwellenstrahlung. Ferner betrifft die Erfindung eine entsprechende Vorrichtung.

Bei der Herstellung verschiedenster Produkte in der industriellen Chemie ist eine Überwachung des Reaktionsfortschrittes chemischer Reaktionen sowohl im Laborwie auch im industriellen Maßstab von großer Bedeutung, um einerseits einen möglichst effizienten Rohstoff- und Energieeinsatz sicherzustellen. Andererseits bietet die Überwachung des Reaktionsfortschrittes die Möglichkeit, im Wege einer umfassenden datengestützten Prozessanalyse ein chemisches Herstellungsverfahren derart zu optimieren, dass u.a. ein gleichbleibend hohe Produktqualität ermöglicht wird. Ein solches Optimierungsverfahren ist in allgemeiner Form beispielsweise in der WO 2019/138120 A1 oder WO 2019/138118 A1 beschrieben.

Bei der Überwachung des Reaktionsfortschrittes von Polyurethan- oder Polyisocyanuratreaktionen ist u.a. die Ermittlung von Reaktionszeiten von entscheidender Bedeutung zur Charakterisierung der Reaktionsablaufes. Dies erfolgt in der Praxis oftmals durch das Laborpersonal. Dabei wird der Startzeitpunkt beispielsweise durch einen Farbumschlag gemessen, während der Zeitpunkt der Abbindung z.B. über einen sogenannten Stäbchentest auf der Schaumoberfläche ermittelt werden kann. Das Verfahren ist dabei erkennbar subjektiv und damit entsprechend schwankungsbehaftet. Eine objektive, jedoch indirekte Ermittlung von Reaktionsumsätzen erfolgt unter anderem über die Messung der Viskosität, der Dielektrizitätskonstante oder der Steighöhe.

Verfahren und Vorrichtungen zur Überwachung des Reaktionsfortschrittes einer Polymerisationsreaktion der eingangs genannten Art sind daneben aus der Patentliteratur und der Praxis bekannt. So beschreibt beispielsweise die EP 2 241 879 A1 ein Verfahren zur Überwachung der Vernetzungsreaktion bei einem duroplastischen Polymer, insbesondere einem Polyurethan. Hierbei wird das Reaktionsgemisch in eine Form gefüllt und mit Mikrowellenstrahlung beaufschlagt. Im Einzelnen wird die Mikrowellenstrahlung zunächst durch einen Resonator geleitet und das von dem Reaktionsgemisch durch den Resonator reflektierte Signal ausgewertet. Dabei wird ausgenutzt, dass gewisse physikalische Kenngrößen des Reaktionsgemisches sich in Abhängigkeit vom Reaktionsfortschritt ändern. Speziell wird der Verlauf der komplexen Permittivität des Reaktionsgemisches als Maß für den Reaktionsfortschritt verwendet. Die komplexe Permittivität kann wiederum aus dem messtechnisch unmittelbar zugänglichen zeitlichen Verlauf des Reflexionskoeffizienten über eine Berechnung der zeitabhängigen Resonanzfrequenz und Gütefaktor des Resonators ermittelt werden. Insgesamt ist nicht erkennbar, dass das beschriebene Verfahren über einen Einsatz im Labormaßstab hinaus nutzbar ist, da für die Produktion von Polymeren relevante Größen, wie Reaktionsstart- oder Abbindezeit nicht ermittelt werden. Ferner ist der Versuchsaufbau, welcher einen separaten Resonator erfordert, zu aufwändig.

Aus der Praxis und aus der DE 10044952 A1 ist ferner ein Messgerät bekannt, mit welchem in einer Schäumreaktion von PUR/PIR-Systemen verschiedene Kenngrößen, wie dielektrischen Polarisation Steighöhe, Temperatur und Druck mittels Ultraschall ermittelt werden können. Eine direkte Messung des PUR/PIR Reaktionsumsatzes ist mit dem Gerät nicht möglich.

Der vorliegenden Erfindung liegt daher die Aufgabe zugrunde, ein Verfahren und eine Vorrichtung zur Überwachung des Reaktionsfortschritts einer Polymerisationsreaktion, insbesondere zur Herstellung von Polyurethanen oder Polyisocyanuraten, anzugeben, welches ohne Aufwand zu realisieren ist und eine einfache Überwachung der für einen industriellen Einsatz relevanten Parameter der Polymerisationsreaktion erlaubt.

Die Aufgabe wird nach einem ersten Aspekt der vorliegenden Erfindung mit einem Verfahren zur Überwachung des Reaktionsfortschritts einer Polymerisationsreaktion, insbesondere zur Herstellung von Polyurethanen oder Polyisocyanuraten, mittels

Mikrowellen- oder Submillimeterwellenstrahlung gelöst, wobei das Verfahren folgende Verfahrensschritte umfasst:
- Bereitstellen einer Fläche zur Aufnahme eines Reaktionsgemisches für eine Polymerisationsreaktion,
- Bereitstellen einer Sendeeinheit und einer Empfangseinheit für Mikrowellen- oder Submillimeterwellenstrahlung, wobei die Fläche bezogen auf den Strahlverlauf zwischen Sendeeinheit und Empfangseinheit angeordnet wird, wobei der Abstand zwischen Sendeeinheit und Empfangseinheit bestimmt wird und wobei die Sendeeinheit und die Empfangseinheit mit einer Auswerteeinheit verbunden sind,
- Aufbringen eines Reaktionsgemisches auf die Fläche,
- wiederholtes Durchstrahlen des Reaktionsgemisches mit FMCW-modulierter Mikrowellen- oder Submillimeterwellenstrahlung mittels der Sendeeinheit,
- Empfangen der Mikrowellen- oder Submillimeterwellenstrahlung nach Durchgang durch das Reaktionsgemisch mittels der Empfangseinheit,
- Bestimmung der Signallaufzeit und/oder der Signalschwächung der Mikrowellen- oder Submillimeterwellenstrahlung von der Sendeeinheit zur Empfangseinheit in der Auswerteeinheit zur Überwachung des Reaktionsfortschrittes der Polymerisationsreaktion.

Die vorliegende Erfindung beruht auf der Erkenntnis, dass bei bekanntem Abstand zwischen Sendeeinheit und Empfangseinheit durch die Messung der zeitlichen Änderung der Signallaufzeit und/oder der Signalschwächung bei mittels im FMCW-Verfahren (Frequency Modulated Continuous Wave) modulierter Mikrowellen- oder Submillimeterstrahlung während des Polymerisationsreaktion gezielt eine Vielzahl reaktionsrelevanter Parameter, die unmittelbar die mit dem erfindungsgemäßen Verfahren überwachte Reaktion charakterisieren, beobachtet werden können. Wie aus dem Stand der Technik bekannt, ändert sich mit fortschreitender Polymerisationsreaktion die Permittivität des Reaktionsgemisches. Dies führt zu Veränderungen der Signallaufzeit, was die vorliegende Erfindung durch Anwendung der FMCW-Technik als Frequenzverschiebung misst und zur Charakterisierung des Reaktionsfortschrittes ausnutzt. In dem sich ergebenden Signalverlauf über die Zeit können die relevanten, den Reaktionsfortschritt charakterisierenden Reaktionszeiten, wie beispielsweise der Startzeitpunkt der Reaktion, der Gelpunkt, der Abbindezeitpunkt und/oder die Klebefreizeit abgelesen werden. Das FMCW-Verfahren ist in der Mikrowellen- und Radartechnik etabliert, so dass geeignete Strahlquellen zur Verfügung stehen. Ein großer Vorteil der FMCW-Technik gegenüber der CW-Technik (Continuous Wave) ist die simultane Ermittlung der Geschwindigkeit, durch die Auswertung der Dopplerverschiebung, und der Entfernung. Für FMCW wird hauptsächlich die lineare Änderungen der Frequenz, sowie die Ausnutzung unterschiedlicher Steigungen verwendet. Hierunter fallen auch die Verwendung der Sägezahn- oder Dreiecksform. Des Weiteren ist die Technik geeignet mehrere Ziele gleichzeitig zu erkennen. Durch geeignete Änderung der Frequenzrampensteigung, können die Ziele eindeutig zugeordnet werden. Für die Mikrowellen- oder Submillimeterstrahlung eignet sich hier ein Frequenzbereich zwischen 10 GHz und 500 GHz.

Nach einer vorteilhaften Ausgestaltung der Erfindung ist vorgesehen, dass die Bestrahlung mit Mikrowellen- oder Submillimeterwellenstrahlung kontinuierlich oder quasi-kontinuierlich erfolgt. Durch eine hohe Wiederholfrequenz der Messung kann der Reaktionsfortschritt praktisch in Echtzeit überwacht, verfolgt und insbesondere die die Reaktion charakterisierenden Zeitpunkte präzise bestimmt werden.

Erfindungsgemäß wird im Rahmen des Verfahrens eine Fläche zur Aufnahme eines Reaktionsgemisches für eine Polymerisationsreaktion bereitgestellt. Die Fläche kann praktisch eine beliebige Form haben. Es muss lediglich sichergestellt sein, dass das Reaktionsgemisch während der Polymerisationsreaktion im Wesentlichen auf der Fläche verbleibt, um ein wiederholtes Durchstrahlen des Reaktionsgemisches mit FMCW-modulierter Mikrowellen- oder Submillimeterwellenstrahlung zu ermöglichen. Beispielsweise kann die Fläche als einfache ebene Fläche oder Platte ausgebildet sein, auf die das Reaktionsgemisch aufgetragen wird. In einer praktischen Anwendung kann es sich bei dem Reaktionsgemisch um eine Mischung aus Polyol und Isocyanat handeln, welche in einer Polyadditionsreaktion zu einem Polyurethanschaum reagieren. Ist die Fläche aus einem für Mikrowellen- oder Submillimeterwellenstrahlung transparenten oder teiltransparenten Material gebildet, kann beispielsweise das Reaktionsgemisch nach dem Auftrag von oben durch die Sendeeinheit mit Mikrowellen- oder Submillimeterwellenstrahlung bestrahlt werden, wobei eine unterhalb der Fläche angeordnete Empfangseinheit die durch das Reaktionsgemisch transmittierte Strahlung empfängt und an die Auswerteeinheit weiterleitet.

Nach einer vorteilhaften Ausgestaltung der Erfindung ist die Fläche zur Aufnahme eines Reaktionsgemisches als Behälter ausgebildet, in welchen das Reaktionsgemisch eingefüllt wird. Der Behälter und das darin befindliche Reaktionsgemisch kann dann in der vorstehend beschriebenen Weise mit Mikrowellen- oder Submillimeterwellenstrahlung beaufschlagt werden. Dabei kann der Behälter beliebige Formen, insbesondere eine einfache rechteckige oder kreisförmige Grundfläche, aufweisen. Die Form sollte jedoch derart gestaltet sein, dass der Behälter aufgrund seiner Geometrie nicht das Ausbreitungsverhalten der Strahlung beeinflusst.

Ist die Fläche wiederum einfach plattenförmig ausgebildet, kann nach einer weiteren Ausgestaltung vorgesehen sein, dass das Reaktionsgemisch mit einer sogenannten Gießharke gleichmäßig in mehreren Spuren auf eine sich unter der Geißharke hinweg bewegende ebene Fläche etwa in Form eines Metallbandes aufgetragen wird. Ebenso kann die Gießharke über die ruhende Fläche bewegt werden. Auf diese Weise können in einem industriellen Produktionsprozess beispielsweise Dämmplatten oder auch Metallverbundelemente produziert werden.

Nach einer besonders vorteilhaften Ausgestaltung der Erfindung ist vorgesehen, dass das Verfahren in Reflexion durchgeführt wird, derart, dass die Mikrowellen- oder Submillimeterwellenstrahlung nach Durchgang durch das Reaktionsgemisch an einer für Mikrowellen- oder Submillimeterwellenstrahlung reflektierenden Oberfläche, insbesondere an einer Metallplatte oder einem anderen geeigneten Reflektor, auf die Empfangseinheit zurückreflektiert wird. Hierdurch wird ein besonders einfach in einen Produktionsprozess integrierbarer Aufbau realisiert, ggf. auch in Form eines nachträglichen Einbaus. Ein besonders einfacher Aufbau wird dadurch realisiert, dass die reflektierende Oberfläche durch die das Reaktionsgemisch tragende Fläche gebildet wird. Im Falle eines das Reaktionsgemisch aufnehmenden Behälters kann die reflektierenden Oberfläche insbesondere durch den Behälterboden gebildet werden. Bevorzugt sind Sende- und Empfangseinheit dabei derart zueinander ausgerichtet, dass das Reaktionsgemisch die Mikrowellen- oder Submillimeterwellenstrahlung zweifach durchstrahlt.

Nach einer weiteren vorteilhaften Ausgestaltung der Erfindung ist vorgesehen, dass im Falle einer Durchführung des Verfahrens in Reflexion die Sendeeinheit und die Empfangseinheit kombiniert in einer Baueinheit angeordnet sind. Hierbei können beispielsweise die Antennen der Sende- und Empfangseinheit in einer Baueinheit angeordnet sein, während der Signalgenerator der Sendeinheit und der Sensor der Empfangseinheit getrennt verbaut sind. Ebenso ist möglich, dass Signalgenerator und Sensor auf einer Platine in der Auswerteeinheit vorgesehen sind.

Das erfindungsgemäße Verfahren eignet sich zur Überwachung des Reaktionsfortschritts beliebiger Polymerisationsreaktionen, soweit sichergestellt ist, dass das Reaktionsgemisch für Mikrowellen- oder Submillimeterwellenstrahlung teilweise transparent ist. Insbesondere eignet es sich zur Überwachung des Reaktionsfortschritts bei Stufenwachstumsreaktionen, d.h. Polykondensations- und Polyadditionsreaktionen. Hierunter fallen rein exemplarisch die Herstellung von Polycarbonat (Polykondensation) oder Polyurethan/Polyisocyanurat (Polyaddition). Wie die Erfinder herausgefunden haben, eignen sich insbesondere Schäumungsreaktionen, speziell Polyurethan- oder Polyisocyanurat-Schäumungsreaktionen. Hier können beispielsweise der Startzeitpunkt, der Gelpunkt, der Abbindezeitpunkt und/oder die Klebefreizeit mit dem erfindungsgemäß Verfahren präzise ermittelt werden. Es lassen sich jedoch auch nichtschäumende Systeme mit dem erfindungsgemäßen Verfahren überwachen.

Nach einem zweiten Aspekt der vorliegenden Erfindung wird die eingangs gestellte Aufgabe gelöst durch eine Vorrichtung zur Überwachung des Reaktionsfortschritts einer Polymerisationsreaktion, insbesondere zur Herstellung von Polyurethanen oder Polyisocyanuraten, mittels Mikrowellen- oder Submillimeterwellenstrahlung, wobei die Vorrichtung umfasst:
- eine Fläche zur Aufnahme eines Reaktionsgemisches für eine Polymerisationsreaktion,
- eine Sendeeinheit, wobei die Sendeeinheit eingerichtet ist, ein Reaktionsgemisch mittels Mikrowellen- oder Submillimeterwellenstrahlung wiederholt zu durchstrahlen,
- eine Empfangseinheit für Mikrowellen- oder Submillimeterwellenstrahlung, wobei die Empfangseinheit eingerichtet ist, die durch das Reaktionsgemisch transmittierte Mikrowellen- oder Submillimeterwellenstrahlung zu empfangen, und
- eine mit der Sendeeinheit und der Empfangseinheit verbundene Auswerteeinheit,
wobei die Vorrichtung gemäß dem Verfahren nach einem der Ansprüche 1 bis 9 eingerichtet ist, um den Reaktionsfortschritt der Polymerisationsreaktion durch Bestimmung der Laufzeit und/oder der Signalschwächung der Mikrowellen- oder Submillimeterwellenstrahlung von der Sendeeinheit zur Empfangseinheit in der Auswerteeinheit zu überwachen.

Für die Vorteile der erfindungsgemäßen Vorrichtung gilt das Vorstehende entsprechend. Insbesondere ist die erfindungsgemäße Vorrichtung einfach aufgebaut und lässt sich geeignet in eine Produktionsanlage für einen Polymerwerkstoff integrieren. Nach einer vorteilhaften Ausgestaltung der Erfindung ist die Fläche zur Aufnahme eines Reaktionsgemisches als Behälter ausgebildet.

Ferner kann vorgesehen sein, dass die Vorrichtung eine für Mikrowellen- oder Submillimeterwellenstrahlung reflektierende Oberfläche umfasst, derart, dass die Mikrowellen- oder Submillimeterwellenstrahlung das Reaktionsgemisch zweifach durchstrahlt.

Darüber hinaus kann vorgesehen sein, die reflektierende Oberfläche durch die Fläche zur Aufnahme des Reaktionsgemisches oder den Boden des Behälters ausgebildet ist.

Nach einer vorteilhaften Ausgestaltung der Erfindung umfasst die Vorrichtung eine kombinierte Sende- und Empfangseinheit für Mikrowellen- oder Submillimeterwellenstrahlung, insbesondere in einer Baueinheit.

Im Folgenden wir die Erfindung durch eine Ausführungsbeispiele darstellende Zeichnung näher erläutert. Es zeigen:
- Fig. 1: Eine Vorrichtung zur Überwachung des Reaktionsfortschritts einer Polymerisationsreaktion zur Herstellung von Polyurethan in einer ersten Ausgestaltung in stark schematisierter Ansicht,
- Fig. 2: Die Vorrichtung der Fig. 1 mit markierten Abständen zwischen der kombinierten Sende-/Empfangseinheit und den weiteren Vorrichtungskomponenten sowie dem Reaktionsgemisch,
- Fig. 3: Die Vorrichtung der Fig. 1 mit markierten Abständen bei weiter fortgeschrittener Polymerisationsreaktion,
- Fig. 4: Die Vorrichtung der Fig. 1 in leicht modifizierter Ausgestaltung mit markierten Abständen,
- Fig. 5: eine Vorrichtung zur Überwachung des Reaktionsfortschritts einer Polymerisationsreaktion zur Herstellung von Polyurethan in einer zweiten Ausgestaltung in stark schematisierter Ansicht,
- Fig. 6: eine Vorrichtung zur Überwachung des Reaktionsfortschritts einer Polymerisationsreaktion zur Herstellung von Polyurethan in einer dritten Ausgestaltung in stark schematisierter Ansicht,
- Fig. 7: eine Vorrichtung zur Überwachung des Reaktionsfortschritts einer Polymerisationsreaktion zur Herstellung von Polyurethan in einer vierten Ausgestaltung in stark schematisierter Ansicht,
- Fig. 8: eine Vorrichtung zur Überwachung des Reaktionsfortschritts einer Polymerisationsreaktion zur Herstellung von Polyurethan in einer fünften Ausgestaltung in stark schematisierter Ansicht,
- Fig. 9: den zeitlichen Verlauf der Magnitude für zwei durchgeführte Messungen an einem ersten PUR/PIR-Reaktionsgemisch gemessen mit einer Vorrichtung gemäß Fig. 1,
- Fig. 10: den zeitlichen Verlauf der Magnitude und des gemessenen Abstandes für ein zweites PUR/PIR-Reaktionsgemisch gemessen mit einer Vorrichtung nach Fig. 5, und
- Fig. 11: den zeitlichen Verlauf der Magnitude und des gemessenen Abstandes für ein drittes PUR/PIR-Reaktionsgemisch gemessen mit einer Vorrichtung nach Fig. 5.

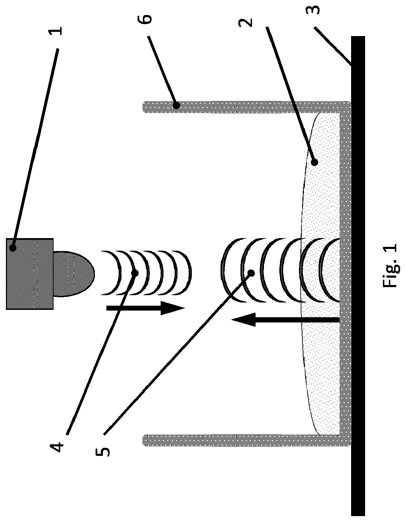

Fig. 1 zeigt eine Vorrichtung zur Überwachung des Reaktionsfortschritts einer Polymerisationsreaktion zur Herstellung von Polyurethan in einer ersten Ausgestaltung in stark schematisierter Ansicht. Die Vorrichtung umfasst eine kombinierte Sende- und Empfangseinheit 1 für Mikrowellen- oder Submillimeterwellenstrahlung, vorliegend im Bereich von 120 GHz. Die kombinierte Sende- und Empfangseinheit 1 für Mikrowellen- oder Submillimeterwellenstrahlung ist mit einer nicht dargestellten Auswerteeinheit, beispielsweise einem Personal Computer oder Laptop verbunden. Unterhalb der kombinierten Sende- und Empfangseinheit 1 ist ein Behälter 6 angeordnet, in welchen eine PUR/PIR-Reaktionsmischung 2 eingefüllt ist, die bereits unter Ausbildung eines Schaumes reagiert. Der das Reaktionsgemisch aufnehmende Behälter 6 seinerseits ist auf einer für Mikrowellen- und Submillimeterwellenstrahlung reflektierenden Oberfläche, vorliegende einer Metallplatte 3 angeordnet. Die von der Sendeeinheit ausgesandte Mikrowellenstrahlung 4 passiert das PUR/PIR Reaktionsgemisch 2 und wird von der reflektierenden Metallplatte 3 reflektiert. Dadurch durchläuft die Mikrowellenstrahlung nochmals das reaktive Gemisch 2, wobei die reflektierte und von der Empfangseinheit empfangene Mikrowellenstrahlung 5 in der kombinierten Sende- und Empfangseinheit 1 in elektrische Signale umgewandelt und an die Auswerteeinheit weitergeleitet wird.

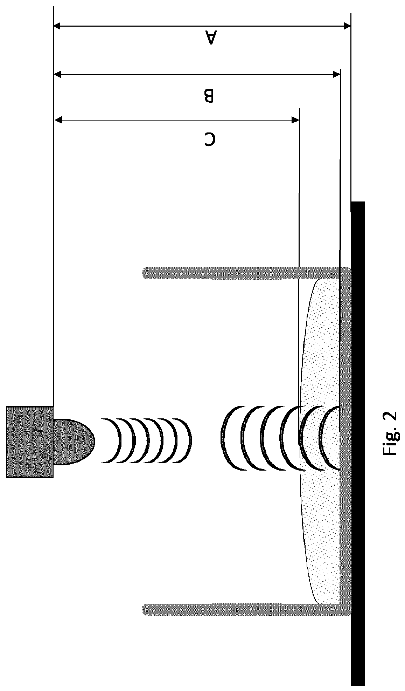

In Fig. 2 sind die für die Messung relevanten Abstände in der Vorrichtung der Fig 1 dargestellt. Der Abstand A stellt hierbei die Distanz zwischen der kombinierten Sende-/Empfangseinheit 1 und der Reflexionsfläche 3 dar. Der Abstand B bezeichnet die Distanz zwischen der Unterseite des Reaktionsgemisches 2, welche eine Grenzschicht darstellt, und der kombinierten Sende-/Empfangseinheit 1. Der Abstand C bezeichnet die Distanz zwischen dem Sensorsystem 1 und der Schäumfront Reaktionsgemisches 2. Durch alle eingezeichneten Grenzflächen wird jeweils eine Reflexionssignal erzeugt, welches von der kombinierten Sende-/Empfangseinheit 1 aufgenommen und in der Auswerteeinheit verarbeitet wird. Durch Einsatz des FMCW-Verfahrens können diese Reflexionssignale aufgelöst werden.

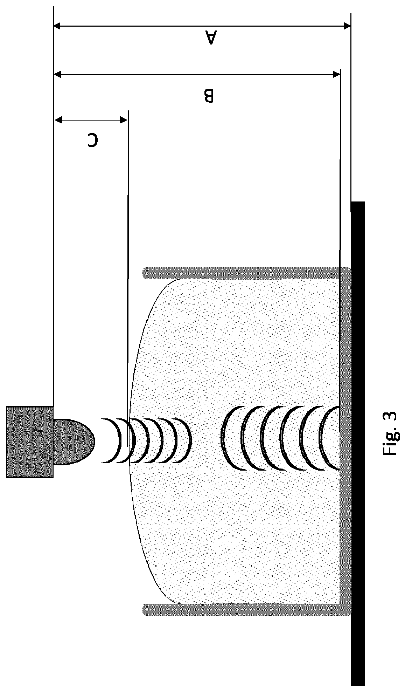

In Fig. 3 ist die aus Fig. 1 und 2 bekannte Vorrichtung mit veränderter Schäumfronthöhe dargestellt. Hier ist die Reaktion im Reaktionsgemisch 2 also deutlich weiter fortgeschritten. Dementsprechend ist in Fig. 3 der Abstand C aufgrund es verringerten Abstandes zwischen der kombinierten Sende-/Empfangseinheit 1 und der Schäumfront stark verkürzt. Die Abstände A (Distanz zwischen kombinierter Sende-/Empfangseinheit 1 und Reflexionsfläche 3) und B (Distanz zwischen kombinierter Sende-/Empfangseinheit 1 und der unteren Grenzschicht des Reaktionsgemisches 2) sind unverändert.

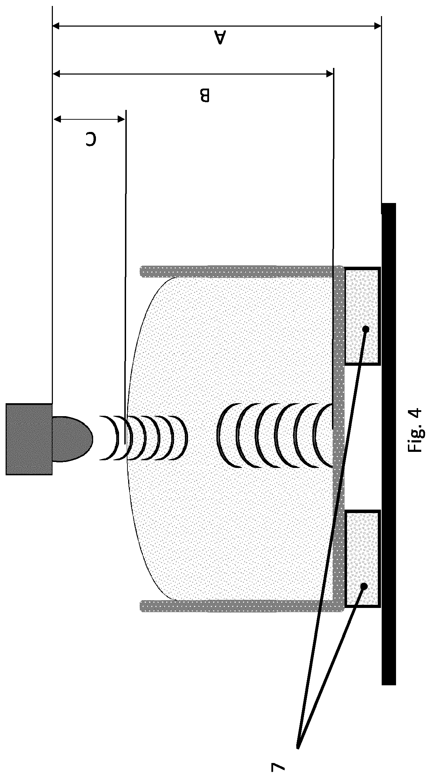

In Fig. 4 ist die aus Fig. 1 bekannte Vorrichtung um zwei Abstandblöcke 7, welche unter dem Behälter 6 platziert sind, ergänzt. Die Abstandsblöcke 7 realisieren einen größeren Abstand zwischen der unteren Grenzschicht des Reaktionsgemisches 2 und der Reflexionsfläche 3, so dass hier eine reduzierte Signalüberlagerung vorliegt. Zusätzlich sind die in Fig. 3 beschreiben Abstände A, B und C dargestellt.

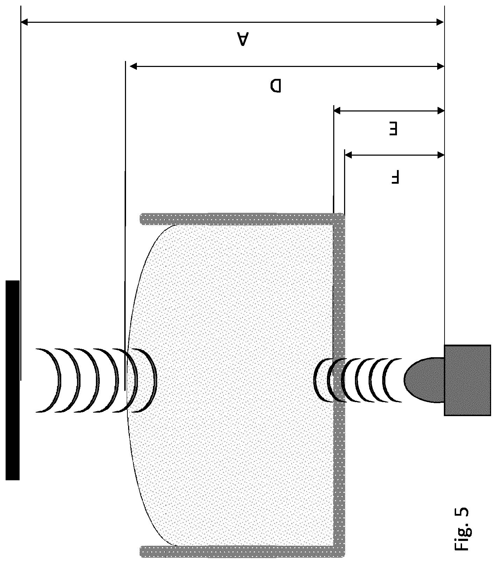

Fig. 5 zeigt eine Vorrichtung zur Überwachung des Reaktionsfortschritts einer Polymerisationsreaktion zur Herstellung von Polyurethan in einer zweiten Ausgestaltung in stark schematisierter Ansicht. In Fig. 5 ist die kombinierte Sende-/Empfangseinheit 1 unterhalb und die Reflexionsfläche 3 oberhalb des offenen Behälters 6 angeordnet. Die vom Sensorsystem 1 ausgehende Mikrowellenstrahlung 4 durchläuft hier das Reaktionsgemisch 2 zunächst von unten nach oben, wird dann an der Reflexionsfläche 3 reflektiert, durchläuft ein zweites Mal das Reaktionsgemisch 2 und wird durch die kombinierte Sende-/Empfangseinheit 1 empfangen und an die Auswerteeinheit weitergeleitet. Der Abstand A stellt dabei die Distanz zwischen dem Sensorsystem 1 und der Reflexionsfläche 3, der Abstand D die Distanz zwischen der kombinierten Sende-/Empfangseinheit 1 und der oberen Grenzschicht des Reaktionsgemisches 2 dar. Der Abstand E bezeichnet die Distanz zwischen kombinierter Sende-/Empfangseinheit 1 und der unteren Grenzschicht des Reaktionsgemisches 2. Der Abstand F wiederum bezeichnet die Distanz zwischen kombinierter Sende-/Empfangseinheit 1 und der Unterseite des Behälters 6.

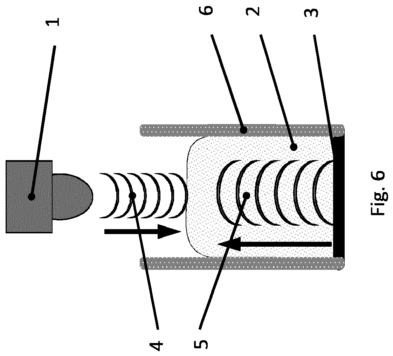

In Fig. 6 ist eine Vorrichtung zur Überwachung des Reaktionsfortschritts einer Polymerisationsreaktion zur Herstellung von Polyurethan in einer dritten Ausgestaltung in stark schematisierter Ansicht dargestellt. Der Behälter 6 hat in dieser Anordnung statt eines für Mikrowellenstrahlung transparenten Bodens eine Reflexionsfläche 3, wodurch eine Grenzflächenreflexion wegfällt. Die ausgehende 4 und die empfangende 5 Mikrowellenstrahlung durchläuft hierbei zweimal das Reaktionsgemisch 2, welches sich in dem offenen Behälter 6 befindet.

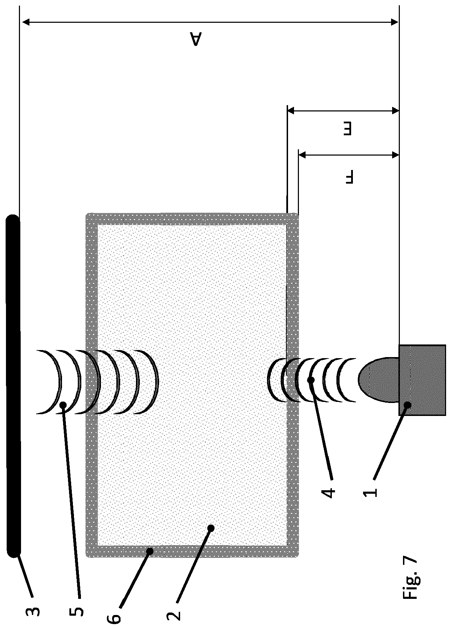

Fig. 7 zeigt eine Vorrichtung zur Überwachung des Reaktionsfortschritts einer Polymerisationsreaktion zur Herstellung von Polyurethan in einer vierten Ausgestaltung in stark schematisierter Ansicht. Hier weist die Vorrichtung einen geschlossenen formgebenden Behälter 6 auf, der vorliegend quaderförmig ist und in dem sich das Reaktionsgemisch 2 befindet. Das Reaktionsgemisch 2 wird hierbei durch die außenliegende kombinierte Sende-/Empfangseinheit 1 derart durchleuchtet, dass die ausgesendete Strahlung 4 an der Reflexionsfläche 3 reflektiert wird und die empfangende Strahlung 5 nochmals durch das Reaktionsgemisch 2 geführt wird. Der Abstand A bezeichnet die Distanz zwischen der kombinierten Sende-/Empfangseinheit 1 und der Reflexionsfläche 3, der Anstand E die Distanz zwischen der kombinierten Sende-/Empfangseinheit 1 und der unteren Grenzschicht des Reaktionsgemisches 2 und der Abstand F die Distanz zwischen der kombinierten Sende-/Empfangseinheit 1 und der Unterseite des geschlossenen Behälters 6.

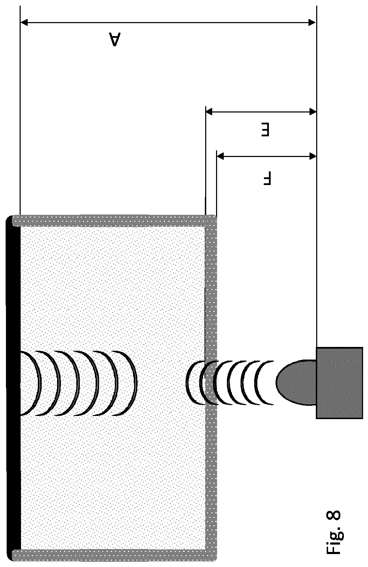

Fig. 8 zeigt eine Vorrichtung zur Überwachung des Reaktionsfortschritts einer Polymerisationsreaktion zur Herstellung von Polyurethan in einer fünften Ausgestaltung, wobei gegenüber der Vorrichtung der Fig. 7 hier die Reflexionsfläche 3 in dem geschlossenen Behälter 6 integriert ist und als Begrenzung für das Reaktionsgemisch 2 fungiert. Der Abstand A bezeichnet wiederum die Distanz zwischen der kombinierten Sende-/Empfangseinheit 1 und der Reflexionsfläche 3, die hier die Oberseite des geschlossenen Behälters 6 ausbildet.

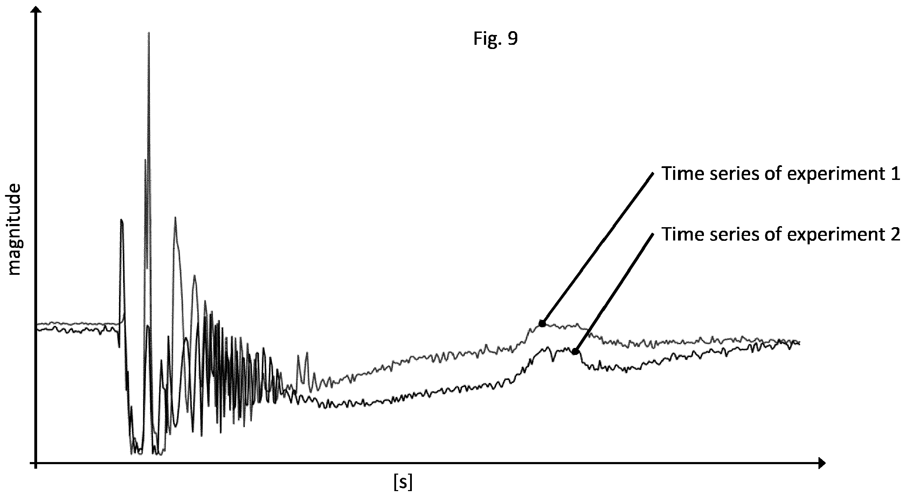

In Fig. 9 ist nun der zeitliche Verlauf der Signalmagnitude für zwei durchgeführte Messungen an einem ersten PUR/PIR-Reaktionsgemisch gemessen mit einer Vorrichtung gemäß Fig. 1 dargestellt. Die beiden Messungen wurden an unterschiedlichen Batches (Chargen) des ersten PUR/PIR-Reaktionsgemisches durchgeführt, wodurch sich sichtbare Abweichungen ergeben. Der Verlauf der Magnitude zeigt charakteristische Merkmale auf, beginnend mit einer konstanten Phase zu Beginn der Messung. Hier befindet sich noch kein Reaktionsgemisch 2 im Behälter 6. Sobald das Reaktionsgemisch 2 im Behälter 6 ist, sinkt die Magnitude stark ab, um dann nach einer kurzen Ruhephase in eine Oszillationsphase überzugehen. Die Frequenz der Oszillation steigt mit der Zeit an und ist allerdings ab etwa der Hälfte wegen Aliasing Effekten nicht mehr korrekt dargestellt. Im letzten Drittel ist nochmals ein langgezogener Peak zu erkennen. Die Magnitude läuft schlussendlich gegen einen Grenzwert. Ist der Grenzwert für ein bestimmtes PUR/PIR-Reaktionsgemisch für einen gegebenen Aufbau bekannt, kann der Fortschritt der Nachreaktion als Quotient aus momentaner und endgültiger Magnitude ermittelt werden.

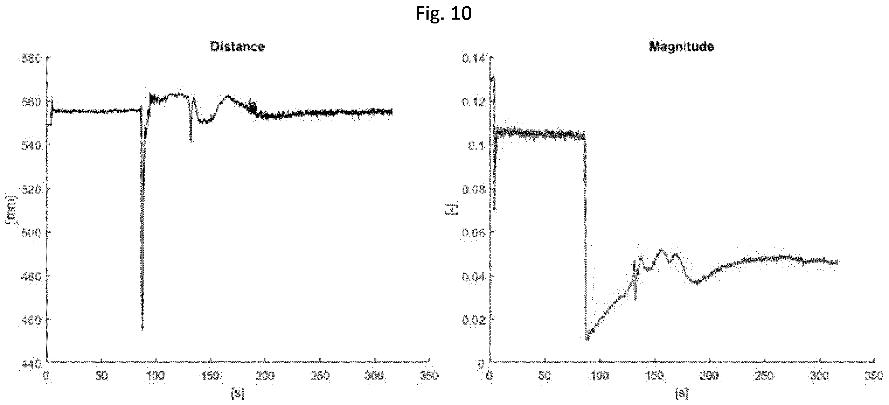

Fig. 10 zeigt den zeitlichen Verlauf der Magnitude und des gemessenen Abstandes für ein zweites PUR/PIR-Reaktionsgemisch gemessen mit einer Vorrichtung nach Fig. 5. Im Einzelnen ist der zeitliche Verlauf der Magnitude und des gemessenen Abstandes als Ergebnis einer FFT-Analyse der Zwischenkreisfrequenz dargestellt. Die Zwischenkreisfrequenz ergibt sich, wie aus der FMCW-Technik an sich bekannt, als Differenzfrequenz zwischen der Frequenz des ausgestrahlten Frequenz-Chirps und des laufzeitverzögert wieder empfangenen Chirps. Auf der linken Seite der Fig. 10 ist der gemessene Abstand dargestellt, der sich vom bekannten wahren Abstand durch die Laufzeitverzögerung infolge der Durchstrahlung des Reaktionsgemisches 2 unterscheidet. Im Einzelnen ist zu erkennen, dass vor Einfüllen des Reaktionsgemisches ein nahezu konstanter Abstand gemessen wird, welcher bei ca. 80 Sekunden stark auf ein Minimum sinkt, um anschließend wieder stark zu steigen. Zwischen 80 und 250 Sekunden durchläuft das Signal einen für dieses Reaktionsgemisch charakteristischen Verlauf. Bei Sekunde 130 ist ein markantes Signalverlaufsmuster zu erkennen, welche mit dem Abbindezeitpunkt des Systems korreliert. Bei der Auswertung erfolgte keine Kompensation der Dopplerverschiebung aufgrund des aufsteigenden PUR/PIR-Reaktionsgemisches.

Der Verlauf des Magnitudensignals (rechte Grafik) ist zunächst bis Sekunde 80 ebenfalls nahezu konstant. Mit Einfüllen des Reaktionsgemisches 2 sinkt die Magnitude stark, um dann einen für dieses reaktive System charakteristischen Verlauf anzunehmen. Auch hier ist bei ca. Sekunde 130 ein markantes Doppelpeakmuster zu erkennen. Im weiteren zeitlichen Verlauf ändert sich die Magnitude noch mehrmals, um ab Sekunde 300 gegen einen Grenzwert zu streben.

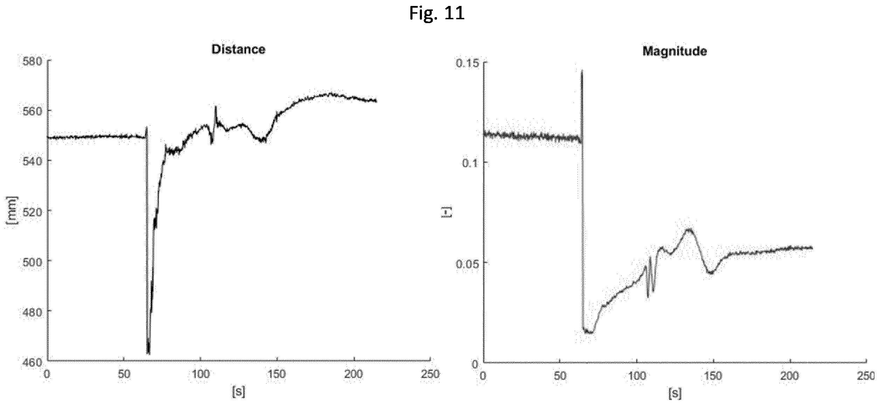

In Fig. 11 ist schließlich der zeitliche Verlauf der Magnitude und des gemessenen Abstandes für ein drittes PUR/PIR-Reaktionsgemisch, welches sich von dem der Fig. 10 unterscheidet, gemessen mit einer Vorrichtung nach Fig. 5 dargestellt. Im Einzelnen ist wiederum der zeitliche Verlauf der Magnitude und des gemessenen Abstandes als Ergebnis einer FFT-Analyse der Zwischenkreisfrequenz dargestellt. Die chemische Reaktion des Reaktionsgemisches ist zur Rezeptur aus Fig. 10 leicht verändert und weist daher einen leicht unterschiedlichen Verlauf auf. Sobald das Reaktionsgemisch in die Form eingefüllt wird, sinkt der Abstand kurzfristig ab um anschließend in einem charakteristischen Verlauf gegen einen Grenzwert zu laufen. Der Verlauf der Magnitude ähnelt der Darstellung der Fig. 10, wobei der sehr schmale Peak mit einem Maximalwert von 0,15 vom Einfüllen des Reaktionsgemisches in den Behälter herrührt und für die Charakterisierung des Reaktionsfortschrittes nicht von Bedeutung ist. Nach dem Einfüllen des Reaktionsgemisches ist die Ruhephase (konstante Magnitude zwischen 60 und 65 Sekunden) etwas ausgeprägter, um dann wieder charakteristisch mit einem Doppelpeak bei Sekunde 120 und weiteren markanten Änderungen gegen einen Grenzwert zu laufen.

## Patentansprüche

1. Verfahren zur Überwachung des Reaktionsfortschritts einer Polymerisationsreaktion, insbesondere zur Herstellung von Polyurethanen oder Polyisocyanuraten, mittels Mikrowellen- oder Submillimeterwellenstrahlung, umfassend folgende Verfahrensschritte:
- Bereitstellen einer Fläche (6) zur Aufnahme eines Reaktionsgemisches (2) für eine Polymerisationsreaktion,
- Bereitstellen einer Sendeeinheit (1) und einer Empfangseinheit (1) für Mikrowellen- oder Submillimeterwellenstrahlung, wobei die Fläche (6) bezogen auf den Strahlverlauf zwischen Sendeeinheit (1) und Empfangseinheit (1) angeordnet wird, wobei der Abstand zwischen Sendeeinheit (1) und Empfangseinheit (1) bestimmt wird und wobei die Sendeeinheit (1) und die Empfangseinheit (1) mit einer Auswerteeinheit verbunden sind,
- Aufbringen eines Reaktionsgemisches (2) auf die Fläche,
- wiederholtes Durchstrahlen des Reaktionsgemisches (2) mit FMCW-modulierter Mikrowellen- oder Submillimeterwellenstrahlung mittels der Sendeeinheit (1),
- Empfangen der Mikrowellen- oder Submillimeterwellenstrahlung nach Durchgang durch das Reaktionsgemisch (2) mittels der Empfangseinheit (1),
- Bestimmung der Laufzeit und/oder der Signalschwächung der Mikrowellen- oder Submillimeterwellenstrahlung von der Sendeeinheit (1) zur Empfangseinheit (1) in der Auswerteeinheit zur Überwachung des Reaktionsfortschrittes der Polymerisationsreaktion.

2. Verfahren nach Anspruch 1,
**dadurch gekennzeichnet, dass**
die Bestrahlung mit Mikrowellen- oder Submillimeterwellenstrahlung kontinuierlich oder quasi-kontinuierlich erfolgt.

3. Verfahren nach Anspruch 1 oder 2,
**dadurch gekennzeichnet, dass**
die Mikrowellen- oder Submillimeterwellenstrahlung eine Frequenz zwischen 10 GHz und 500 GHz aufweist.

4. Verfahren nach einem der Ansprüche 1 bis 3,
**dadurch gekennzeichnet, dass**
die Fläche zur Aufnahme eines Reaktionsgemisches als Behälter (6) ausgebildet ist.

5. Verfahren nach einem der Ansprüche 1 bis 4,
**dadurch gekennzeichnet, dass**
das Verfahren in Reflexion durchgeführt wird, derart, dass die Mikrowellen- oder Submillimeterwellenstrahlung nach Durchgang durch das Reaktionsgemisch (2) an einer für Mikrowellen- oder Submillimeterwellenstrahlung reflektierenden Oberfläche (3), insbesondere an einer Metallplatte, auf die Empfangseinheit zurückreflektiert wird.

6. Verfahren nach Anspruch 5,
**dadurch gekennzeichnet, dass**
die Mikrowellen- oder Submillimeterwellenstrahlung das Reaktionsgemisch (2) zweifach durchstrahlt.

7. Verfahren nach Anspruch 5 oder 6,
**dadurch gekennzeichnet, dass**
die Sendeeinheit und die Empfangseinheit kombiniert in einer Baueinheit (1) angeordnet sind.

8. Verfahren nach einem der Ansprüche 1 bis 7,
**dadurch gekennzeichnet, dass**
die Polymerisationsreaktion eine Schäumungsreaktion, insbesondere Polyurethan- oder Polyisocyanurat-Schäumungsreaktion, ist.

9. Verfahren nach einem der Ansprüche 1 bis 8,
**dadurch gekennzeichnet, dass**
der Startzeitpunkt, der Gelpunkt, der Abbindezeitpunkt und/oder die Klebefreizeit der Polymerisationsreaktion bestimmt wird.

10. Vorrichtung zur Überwachung des Reaktionsfortschritts einer Polymerisationsreaktion, insbesondere zur Herstellung von Polyurethanen oder Polyisocyanuraten, mittels Mikrowellen- oder Submillimeterwellenstrahlung, umfassend:
- eine Fläche (3, 6) zur Aufnahme eines Reaktionsgemisches für eine Polymeri sationsreaktion,
- eine Sendeeinheit (1), wobei die Sendeeinheit (1) eingerichtet ist, ein Reaktionsgemisch (2) mittels Mikrowellen- oder Submillimeterwellenstrahlung wiederholt zu durchstrahlen,
- eine Empfangseinheit (1) für Mikrowellen- oder Submillimeterwellenstrahlung, wobei die Empfangseinheit (1) eingerichtet ist, die durch das Reaktionsgemisch (2) transmittierte Mikrowellen- oder Submillimeterwellenstrahlung zu empfangen, und
- eine mit der Sendeeinheit (1) und der Empfangseinheit (1) verbundene Auswerteeinheit,
wobei die Vorrichtung gemäß dem Verfahren nach einem der Ansprüche 1 bis 9 eingerichtet ist, um den Reaktionsfortschritt der Polymerisationsreaktion durch Bestimmung der Laufzeit und/oder der Signal schwächung der Mikrowellen- oder Submillimeterwellenstrahlung von der Sendeeinheit zur Empfangseinheit in der Auswerteeinheit zu überwachen.

11. Vorrichtung nach Anspruch 10 oder 11,
**dadurch gekennzeichnet, dass**
die Fläche zur Aufnahme eines Reaktionsgemisches als Behälter (6) ausgebildet ist.

12. Vorrichtung nach Anspruch 10 oder 11,
**dadurch gekennzeichnet, dass**
die Vorrichtung sowie eine für Mikrowellen- oder Submillimeterwellenstrahlung reflektierende Oberfläche (3) umfasst, derart, dass die Mikrowellen- oder Submillimeterwellenstrahlung das Reaktionsgemisch zweifach durchstrahlt.

13. Vorrichtung nach Anspruch 12,
**dadurch gekennzeichnet, dass**
die reflektierende Oberfläche (3) durch die Fläche (6) zur Aufnahme des Reaktionsgemisches oder den Boden des Behälters (6) ausgebildet wird.

14. Vorrichtung nach Anspruch 12 oder 13,
**dadurch gekennzeichnet, dass**
die Vorrichtung eine kombinierte Sende- und Empfangseinheit (1) für Mikrowellen- oder Submillimeterwellenstrahlung, insbesondere in einer Baueinheit, umfasst.
